(19) Europäisches Patentamt
European Patent Office
Office européen des brevets

(11) EP 2 643 007 B1

(12) **EUROPEAN PATENT SPECIFICATION**

(45) Date of publication and mention of the grant of the patent:
**10.08.2016 Bulletin 2016/32**

(21) Application number: **11842716.0**

(22) Date of filing: **24.11.2011**

(51) Int Cl.:
*A61K 36/355* (2006.01) *A61K 31/19* (2006.01)
*A61K 31/185* (2006.01) *A61P 1/08* (2006.01)
*A61P 1/04* (2006.01) *A61K 45/06* (2006.01)
*A61K 31/216* (2006.01) *A61K 31/222* (2006.01)
*A61K 31/232* (2006.01) *A23L 29/00* (2016.01)

(86) International application number:
**PCT/KR2011/009027**

(87) International publication number:
**WO 2012/070890 (31.05.2012 Gazette 2012/22)**

# (54) PHARMACEUTICAL COMPOSITION COMPRISING EXTRACT OF LONICERA JAPONICA FOR PREVENTION AND TREATMENT OF GASTROESOPHAGEAL REFLUX DISEASE

PHARMAZEUTISCHE ZUSAMMENSETZUNG MIT EINEM EXTRAKT AUS LONICERA JAPONICA ZUR PRÄVENTION UND BEHANDLUNG VON REFLUXÖSOPHAGITIS

COMPOSITION PHARMACEUTIQUE COMPORTANT UN EXTRAIT DE LONICERA JAPONICA POUR PRÉVENIR ET TRAITER LE REFLUX GASTRO-OESOPHAGIEN PATHOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2010 KR 20100117984**

(43) Date of publication of application:
**02.10.2013 Bulletin 2013/40**

(73) Proprietor: **Green Cross WellBeing Corporation**
**Seongnam-si, Gyeonggi-do, 13595 (KR)**

(72) Inventors:
- **YOO, Young-Hyo**
  **Seoul 135-506 (KR)**
- **KIM, Jeom Yong**
  **Seoul 137-949 (KR)**
- **KIM, Sun Ok**
  **Seoul 134-813 (KR)**
- **KIM, Joo Young**
  **Suwon-city**
  **Gyeonggi-do 442-703 (KR)**
- **PARK, Sun Kyu**
  **Seoul 138-755 (KR)**
- **JANG, Min Jung**
  **Sungnam-city**
  **Gyeonggi-do 462-120 (KR)**

(74) Representative: **Beckmann, Claus et al**
**Kraus & Weisert**
**Patentanwälte PartGmbB**
**Thomas-Wimmer-Ring 15**
**80539 München (DE)**

(56) References cited:
**WO-A1-2010/083968 WO-A2-2011/071296**
**WO-A2-2012/105798 KR-B1- 100 878 436**
**US-A1- 2007 111 955**

- **KU SAE-KWANG ET AL: "Effect of Lonicerae Flos extracts on reflux esophagitis with antioxidant activity.", WORLD JOURNAL OF GASTROENTEROLOGY : WJG 14 OCT 2009, vol. 15, no. 38, 14 October 2009 (2009-10-14), pages 4799-4805, XP55103217, ISSN: 1007-9327**
- **D. TANG ET AL.: 'Rapid and simple method for screening of natural antioxidants from chinese herb Flos Lonicerae Japonicae by DPPH-HPLC-DAD-TOF/ MS' JOURNAL OF SEPERATION SCIENCE vol. 31, 2008, pages 3519 - 3526**
- **S.-K. KU ET AL.: 'Effect of Lonicerae Flos extracts on reflux esophagitis with antioxidant activity' WORLD JOURNAL OF GASTROENTEROLOGY vol. 15, no. 38, 14 October 2009, pages 4799 - 4805**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- B.-I. LEE ET AL.: 'Anti-ulcerogenic effect HPLC analysis of the caffeoylquinic acid-rich extract from Ligularia stenocephala' BIOLOGICAL & PHARMACEUTICAL BULLLETIN vol. 33, no. 3, March 2010, pages 493 - 497

## Description

### Technical Field

The present invention relates to a composition for use in treating or preventing gastroesophageal reflux disease, which includes a Lonicerae Flos extract or fraction having a therapeutic effect on gastroesophageal reflux disease injury. More particularly, the present invention relates to a pharmaceutical composition and a health care food composition for treating or preventing gastroesophageal reflux disease, which includes a Lonicerae Flos extract or fraction containing 3,5-di-O-caffeoylquinic acid and 4,5-di-O-caffeoylquinic acid, as active ingredients, wherein these materials are highly therapeutically effective on gastroesophageal reflux disease and do not show cytotoxicity.

### Background Art

In general, gastroesophageal reflux disease is a disease causing various clinical symptoms (such as brash, epigastric pain) and a change in a mucous membrane due to stomach contents (acid and pepsin) flowing backward into the esophagus. As a time of exposure to acid increases, a serious lesion occurs, resulting in chronic progress.

Typical gastroesophageal reflux disease is mainly caused by the esophagus's excessive exposure to acid and pepsin. On the other hand, it is known that non-erosive reflux disease shows a normal range of exposure to acid and pepsin but is caused by the esophagus's abnormal over-sensitiveness to acid and pepsin.

As an initial therapeutic agent for gastroesophageal reflux disease, a histamine-2 receptor antagonist (H2RA), that is, an acid secretion inhibitor, was used. However, this caused a high frequency of relapse. Furthermore, a therapeutic effect for serious esophagitis was unsatisfactory. Then, a proton pump inhibitor (PPI) having a high acid secretion inhibiting effect has been widely used because it proved good in view of cost-effectiveness.

However, even when the PPI is used, the relapse rate is high. Furthermore, there is a problem in that the PPI has to be taken for a long time. In actuality, it has been reported that after application of the PPI for about 8 weeks, gastroesophageal reflux disease is treated, but within 1 year after the discontinuance of drug application, 50 ~ 80% of patients relapse into the disease. Also, it has been continuously reported that when the PPI is taken for a long time in order to treat gastroesophageal reflux disease, a neuroendocrine cell tumor is caused. Furthermore, in 2010, the US FDA reported that when a PPI-based drug is taken for a long time (e.g., for a year or more) or is taken with a high dosage, a risk of fracture may be increased. Accordingly, it is urgently required to develop a novel therapeutic agent for gastroesophageal reflux disease, which is highly effective in treatment of gastroesophageal reflux disease and harmless to the human body even in long-term administration.

Lonicerae Flos is a flower of *Lonicera japonica* Thunb of Caprifoliaceae, and is used for diuresis, stomach strengthening, arthritis, pyodermatitis, and bronchitis in oriental medicine and folk remedies. It is reported that contents of Lonicerae Flos include tannin, inositol, sterol, chlorogenic acid, isochlogenic acid, etc., and it is known that Lonicerae Flos includes a flavonoid content such as luteolin, apigenin, luteolin-7-O-rhamnoglucoside, quercetin, etc. It was reported that the flavonoid content of Lonicerae Flos has an anti-inflammatory effect (Lee, S. J.; Arch. Pharm. Res. 16, 25, 1993) and an anti-mutagen effect.

In research on Lonicerae Flos, Kang Ok-hee (researcher on pharmacological action of Lonicerae Flos) reported that a methanol extract of Lonicera japonica has an anti-bacterial effect on gram positive bacillus and gram negative bacillus of edema. Lee, et al. reported that through a test on ear edema and sole edema causing carrageenin of a mouse, a butanol fraction of Lonicerae Flos improves the sole edema dependently on the dosage although its effect is lower than prednisolone. (S. J. Lee,; Phytotherapy research. 12, 445-447, 1998) Also, Tae, et al. reported that a water extract of Lonicera japonica flower shows an anti-inflammatory effect by inhibiting activity of NFκB p65, and degradation of IκBα in rat liver sepsis induced by LPS (Lipopolysaccharide).(Tae, J, Han,; Clin Chim Acta. 330(1-2), 165-171, 2003).

Ku, et al. has recently found that a water extract of Lonicera japonica has an effect for inhibiting damage to a mucous membrane of the esophagus in a gastroesophageal reflux disease rat model (Ku, S. K.; World J Gastroenterol. 15(38): 4799-4805, 2009). Also, the inventors of the present invention disclosed Korean Registered Patent No. 10-0878436, titled "Pharmaceutical composition comprising extract of Lonicera japonica for prevention and treatment of digestive ulcer , in which a Lonicera japonica extract shows a therapeutic effect on gastritis • astric ulcer.

The inventors of the present invention found that a specific fraction of Lonicerae Flos is highly effective in gastroesophageal reflux disease during development of a gastritis gastric ulcer therapeutic agent including a Lonicerae Flos extract, and then found that the specific fraction shows a higher therapeutic effect on gastroesophageal reflux disease than the Lonicerae Flos water extract previously researched by Ku. Accordingly, they found that a Lonicerae Flos fraction or a compound isolated therefrom not only is very effective in treatment of gastroesophageal reflux disease, but also shows an effect of protection and regeneration on esophagus mucous membrane cells through an inflammation inhibiting effect and an antioxidant effect of an esophagus mucous membrane. Such an effect reduces a high relapse rate, that is, a problem of conventional therapeutic agents (a PPI and a H2RA), and furthermore shows a preventive

effect on gastroesophageal reflux disease. Thus, they completed this invention by finding out that a composition including the fraction or the compound as an active ingredient can be used as a drug and a health functional food for treatment or prevention of gastroesophageal reflux disease.
J. Sep. Sci. 2008, 31, 3519-3526 discloses a rapid and simple method for screening and identification of natural antioxidants of Flos Lonicerae Japonicae derived from the flower buds of *Lonicera japonica.*

## Disclosure of Invention

### Technical Problem

An object of the present invention is to provide a pharmaceutical composition for treating or preventing gastroesophageal reflux disease, which includes a Lonicerae Flos extract, a Lonicerae Flos fraction, or the compound represented by Formula 1 or 2, as an active ingredient.

[Formula 1]

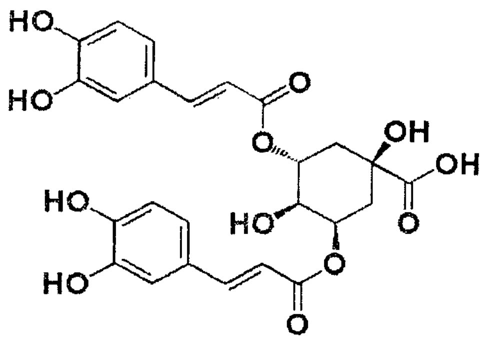

[Formula 2]

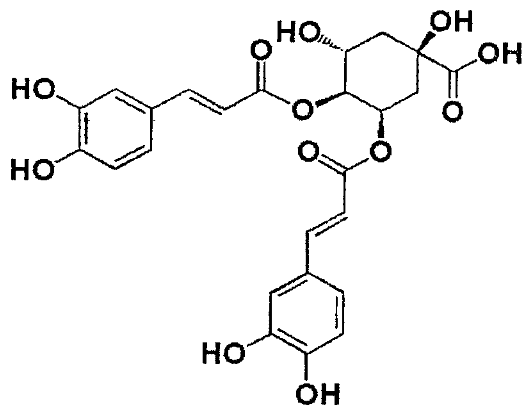

Another object of the present invention is to provide a health care food composition for preventing or improving gastroesophageal reflux disease, which includes a Lonicerae Flos extract, a Lonicerae Flos fraction, or the compound represented by Formula 1 or 2, as an active ingredient.

### Solution to Problem

In order to achieve the above objects, the present invention provides a pharmaceutical composition for use in treating or preventing gastroesophageal reflux disease, the composition comprising, as active ingredients, 3,5-di-O-caffeoylquinic acid (Formula 1) and 4,5-di-O-caffeoylquinic acid (Formula 2) as a C1-C4 alcohol extract or a 50-100% C1 ~ C4 alcohol aqueous solution extract of Lonicerae Flos, or as a ethyl acetate or butanol fraction of said extract

[Formula 1]

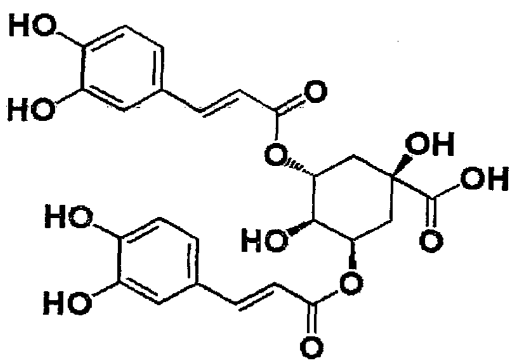

[Formula 2]

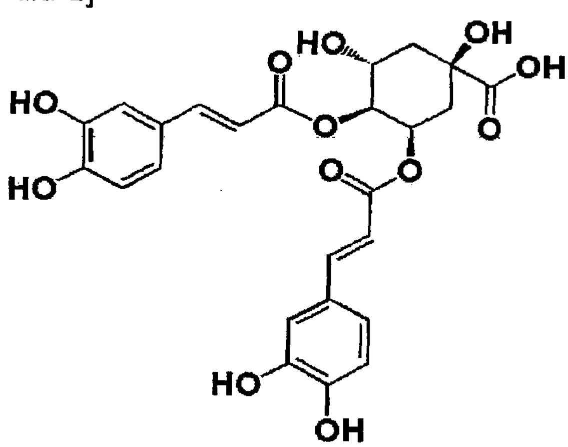

and wherein in the composition the content of 3,5-di-O-caffeoylquinic acid (Formula 1) is 2% or more in relation to the extract or fraction and the content of 4,5-di-O-caffeoylquinic acid (Formula 2) is 1% or more in relation to the extract or fraction.

In accordance with an aspect of the present invention, there is provided a health care food composition for use in improving or preventing gastroesophageal reflux disease, the composition comprising, as active ingredients, 3,5-di-O-caffeoylquinic acid (Formula 1) and 4,5-di-O-caffeoylquinic acid (Formula 2) as a C1-C4 alcohol extract or a 50-100% C1-C4 alcohol aqueous solution extract of Lonicerae Flos, or as a ethyl acetate or butanol fraction of said extract,

[Formula 1]

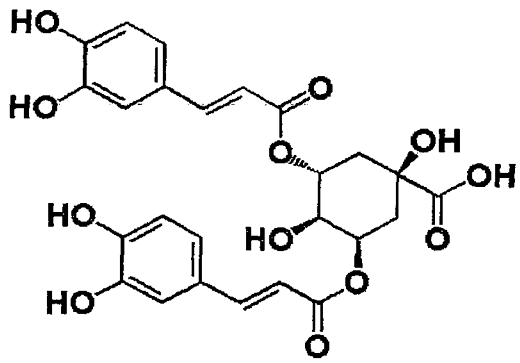

[Formula 2]

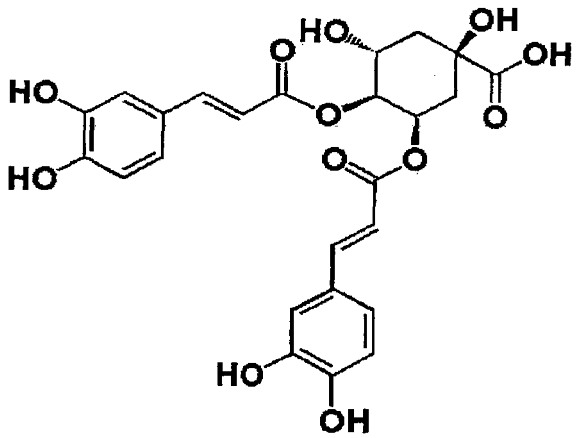

and wherein in the composition the content of 3,5-di-O-caffeoylquinic acid (Formula 1) is 2% or more in relation to the extract or fraction and the content of 4,5-di-O-caffeoylquinic acid (Formula 2) is 1% or more in relation to the extract or fraction.

Further described is a pharmaceutical composition for treatment and prevention of gastroesophageal reflux disease, in which the Lonicerae Flos extract/fraction or the compound represented by Formula 1 or 2 is used alone, or used in combination with other materials currently clinically used as therapeutic agents for gastroesophageal reflux disease, such as H2 receptor antagonist (H2RA) drugs (e.g., cimetidine, ranitidine, nizatidine, famotidine) or proton-pump inhibitor (PPI) drugs (e.g., omeprazol, pantoprazole, lansoprazole, revaprazan) so as to achieve a better therapeutic effect for gastroesophageal reflux disease.

Hereinafter, the present invention will be described in detail. Subject-matter which is not encompassed by the scope of the claims does not form part of the claimed invention.

The extract of Lonicerae Flos, according to the present invention, may be extracted from Lonicerae Flos or dried Lonicerae Flos. The Lonicerae Flos may be wild or cultured. The extract of Lonicerae Flos, according to the present invention, may be extracted through a conventional extraction method known in the art, including a method using an extracting apparatus (such as supercritical extraction, room temperature extraction, high temperature extraction, high pressure extraction, or ultrasonic extraction) and a method using adsorbent resin (such as XAD and HP-20). Preferably, the extract may be obtained through dissolution/reflux extraction or room temperature extraction, but the present invention is not limited thereto. The number of times of extraction preferably ranges from 1 to 5, and more preferably is 3, but the present invention is not limited thereto.

The extract is obtained by using a C1 to C4 alcohol, and is most preferably methanol, ethanol, butanol or a 50~100% alcohol aqueous solution thereof.

As one example, the inventive Lonicerae Flos extract may be obtained by the steps of: grinding dried Lonicerae Flos into an appropriate size, introducing the ground dried Lonicerae Flos into an extraction vessel, adding an extraction solvent thereto, carrying out reflux-extraction while heating the solvent, leaving the resultant product for a predetermined time, and filtering the product through filter paper, etc. so as to provide an alcohol extract. The extraction time preferably ranges from 2 to 12 hours, and more preferably ranges from 3 to 6 hours. Then, concentration or freeze-drying may be additionally carried out.

The fraction of Lonicerae Flos, according to the present invention, may be obtained by independently or sequentially carrying out systematic fractionation of the Lonicerae Flos extract by using ethyl acetate, and butanol. Furthermore, the present description provides a pharmaceutical composition for use in the treatment and prevention of gastroesophageal reflux disease, which includes, as active ingredients, 3,5-di-O-caffeoylquinic acid represented by Formula 1, and 4,5-di-O-caffeoylquinic acid represented by Formula 2.

The compound represented by Formula 1 or 2 may be prepared by the steps of: adding water, a C1-C4 alcohol,

or a mixture thereof to Lonicerae Flos so as to obtain a Lonicerae Flos extract (step 1): suspending the Lonicerae Flos extract obtained from the step 1 in water, and fractionating the extract by ethyl acetate or butanol so as to provide a fraction (step 2): and purifying the fraction obtained from the step 2 by silica gel chromatography so as to separate and purify the compound represented by Formula 1 or 2 (step 3).

Hereinafter, respective steps of the preparation method according to the present disclosure will be described in more detail. First, the step 1 in the inventive method is to obtain a Lonicerae Flos extract by an extraction solvent. A dried Lonicerae Flos is ground into an appropriate size and introduced into an extraction vessel. A solvent may be any one or a combination selected from the group including C1 to C4 alcohol, a 50~100% C1 to C4 alcohol aqueous solution and is preferably methanol, ethanol, butanol or a 50~100% alcohol aqueous solution thereof.

The Lonicerae Flos is subjected to ultrasonic extraction at 60°C for 6 hours, and the resultant product is filtered through filter paper, etc. so as to provide the inventive Lonicerae Flos extract.

Then, the step 2 is to obtain a fraction by fractionating the Lonicerae Flos extract obtained from step 1 by a solvent having a different polarity. As the solvent, ethyl acetate or butanol are used. Next, the step 3 may be to purify the fraction obtained from the step 2 by silica gel chromatography so as to separate and purify a compound represented by Formula 1 or 2. The silica gel chromatography may be carried out by using a size-exclusion chromatography column, and may be preferably carried out by a column containing HP-20. The fraction obtained from the step 2 is purified through silica gel chromatography by using a HP-20 column (mobile phase: ethanol). Herein, the obtained fraction may be purified by high-performance liquid chromatography so as to separate the compound represented by Formula 1 or 2.

The high-performance liquid chromatography may be carried out by using, as a developing solvent, a mixed solvent of water and acetonitrile with a concentration gradient of acetonitrile (0 to 5 vol%; from 5 to 10 vol%).

Herein, the flow rate of the mobile phase ranges from 2 to 15 mL/min.

The inventive Lonicerae Flos fraction or the compound represented by Formula 1 or 2 may be used for treatment or prevention of gastroesophageal reflux disease. Their effect on the treatment or prevention of gastroesophageal reflux disease will be described based on specific experimental results.

In order to determine the therapeutic effect on acute/chronic gastroesophageal reflux disease by the inventive Lonicerae Flos fraction or the compound represented by Formula 1 or 2, the method for measuring the therapeutic effect on gastroesophageal reflux disease (Nakamura K et al; Jpn. J. Pharmacol., 32(3), 445-456, 1982: Okabe S, et al; Jpn. J. Pharmacol., 69(4), 317-323, 1995) was applied to the present experiments.

The pharmaceutical composition for treatment or prevention of gastroesophageal reflux disease, which includes, as an active ingredient, the inventive Lonicerae Flos fraction or the compound represented by Formula 1 or 2, may be formulated into various oral/parenteral dosage forms below.

A preparation for oral administration includes, for example, tablet, pill, hard/soft capsule, liquid, suspension, emulsion, syrup, granule, etc. The preparation may include not only the active ingredient, but also at least one conventionally used diluent or excipient, such as a filler, an extender, a wetting agent, a disintegrating agent, a slip modifier, a binding agent, and a surfactant. As the disintegrating agent, agar, starch, alginic acid or sodium salt thereof, anhydrous dibasic calcium phosphate salt, etc. may be used, as the slip modifier, silica, talc, stearic acid or magnesium salt or calcium salt thereof, polyethylene glycol, etc. may be used, as the binding agent, magnesium, aluminum silicate, starch paste, gelatin, methyl cellulose, sodium carboxymethyl cellulose, polyvinylpyrrolidine, low-substituted hydroxypropylcellulose, etc. may be used, and as the diluent, glycine, etc. may be used. In some cases, conventionally known additives such as an effervescent mixture, an absorbent, a colorant, a flavouring agent, and a sweetening agent may be used.

Also, the pharmaceutical composition for treatment or prevention of gastroesophageal reflux disease, which includes, as active ingredients, the compounds represented by Formula 1 or 2, may be parenterally administered through subcutaneous injection, intravenous injection, intramuscular (breast) injection. Herein, for formulation into a parenteral administration preparation or the compounds represented by Formula 1 or 2, may be mixed with a stabilizer or a buffer in water, and then prepared as a liquid or a suspension. The resultant liquid or suspension may be prepared as an ample or vial form of a dosage unit.

The composition may be sterilized or may contain an antiseptic, a stabilizer, a wettable powder or an emulsifier, a salt for osmotic pressure adjustment, an adjuvant such as a buffer, and therapeutically effective other materials. Also, the composition may be formed into a preparation through a conventional method such as mixing, granulating or coating.

When the pharmaceutical composition for treatment or prevention of gastroesophageal reflux disease, which includes, as active ingredients, the compounds represented by Formula 1 or 2, is formed into a preparation with a unit dose, it is preferable that the composition includes, as the active ingredient, the Lonicerae Flos fraction containing the compound represented by Formula 1 and 2, in a unit dose of about 0.1 to 1500 mg. The dosage is based on a doctor s prescription according to a patient's weight, age, disease particularity, and disease severity.

However, in general, the composition may be orally or parenterally administered to an adult at approximately 0.1 ~ 1000 mg a day according to administration frequencies and intensities. When the composition is intramuscularly or intravenously administered to an adult, it is sufficient that the total dosage divided into multiple doses ranges from 0.1 to 300 mg per day. Meanwhile, for some patients, preferably, a higher daily dosage is required.

The compounds represented by Formula 1 or 2 may be added to a health care/functional food, such as a food or a drink, for the purpose of treatment or prevention of gastroesophageal reflux disease. In this case, the compounds represented by Formula 1 or 2, used as a food additive, may be added in an amount of 0.01 ~ 30wt%, preferably of 0.1~10 wt% with respect to the total amount of raw materials. The amount of an active ingredient to be mixed may be appropriately determined according to the purpose of the use. In a long-term ingestion for the purpose of health care and hygiene or for the purpose of health regulation, the amount may be less than the above mentioned range. However, since there is no problem in view of stability, the active ingredient may be used in an amount greater than the range. The compounds represented by Formula 1 or 2 may used together with other foods or other food compositions, and may be appropriately used according to a conventional method.

There is no specific limitation in the kind of the food. Examples of a food to be added with the fraction or the compound represented by Formula 1 or 2 may include meat, sausage, bread, chocolate, candies, snack, pizza, ramen, other noodles, gums, dairy products including ice cream, various kinds of soups, drink, health drink, alcohol drink, vitamin-mixed formulation, etc., and all kinds of health care foods in their conventional meaning.

In the present invention, as a food preservative additive, various flavoring agents or natural carbohydrate may be used. The natural carbohydrate includes monosaccharide (such as glucose, fructose), disaccharide (such as maltose, sucrose), polysaccharide (such as dextrin, cyclodextrin), and sugar alcohol (such as xylitol, sorbitol, erythritol). As a sweetening agent, a natural sweetening agent such as thaumatin, and stevia extract, or a synthetic sweetening agent such as saccharin, and aspartame may be used. The natural carbohydrate is included in an amount of generally about 0.01~0.04 parts by weight, preferably of 0.02~0.03 parts by weight with respect to 100 parts by weight of the inventive composition.

Besides the above mentioned additives, the inventive composition may contain various nutrients, vitamins, an electrolyte, a flavoring agent, a coloring agent, pectic acid and its salt, alginic acid and its salt, organic acid, a protective colloid thickner, a pH modifier, a stabilizer, an antiseptic, glycerin, alcohol, a carbonation agent for carbonated beverage, etc. They may be used alone or in combination. The additives are generally included in an amount of 0.01 ~0.1 parts by weight with respect to 100 parts by weight of the inventive composition, but the ratio of the additives is not particularly important.

**Advantageous Effects of Invention**

The inventive composition including, as active ingredients, a 3,5-di-O-caffeoylquinic acid represented by Formula 1, and 4,5-di-O-caffeoylquinic acid represented by Formula 2, shows a similar or greater effect on gastroesophageal reflux disease without side effects, as compared to that of a conventional therapeutic agent. Also, the composition not only inhibits inflammation of the esophagus' mucous membrane, but also protects the esophagus' mucous membrane cells, thereby reducing a relapse rate of esophagitis.

The compounds represented by Formula 1 or 2 may be usefully utilized for treatment or prevention of gastroesophageal reflux disease.

Also, the inventive extract containing the compounds represented by Formula 1 and 2 may be used alone, or used in combination with other materials currently clinically used as therapeutic agents for gastroesophageal reflux disease, such as H2 receptor antagonist (H2RA) drugs (e.g., cimetidine, ranitidine, nizatidine, famotidine) or proton-pump inhibitor (PPI) drugs (e.g., omeprazol, pantoprazole, lansoprazole, revaprazan) so as to achieve a better therapeutic effect for gastroesophageal reflux disease.

In the following experimental examples 1 to 6 as disclosed in paragraphs [46] to [74] only the compositions of examples 1-5 as disclosed in paragraphs [79] to [86] fit in the scope of the claims.

<Experimental Example 1: Test of the effect of a Lonicerae Flos extract on gastroesophageal reflux disease >

In order to determine the therapeutic effect of the inventive Lonicerae Flos extract, the inventive Lonicerae Flos fraction, or the compound represented by Formula 1 or 2 in a rat model of gastroesophageal reflux disease, Sprauge-Dawley(SD)-based male rats aged 7 weeks were fasted for 24 hours and supplied with water in a sufficient amount. The rats were weighed, and were orally administered with omeprazole (Sigma-Aldrich) as a control drug and with Lonicerae Flos extract/fraction as a test drug, which are suspended in a 0.5% CMC (Carboxymethyl cellulose) solution, one hour before induction of reflux. For reflux induction, the abdomen of the rat was opened, and a pylorus region between stomach and duodenum was ligated. Then, a stomach-esophagus sphincter between esophagus and stomach was injured by longitudinal incision of 1cm so that gastric acid can be flowed backward due to abnormal relaxation of the sphincter. Also, a transition zone between forestomach and glandular portion was ligated by thread, and the abdomen was sutured by cotton thread. 6 hours after the completion of the operation, inhalation anesthesia was performed, and the esophagus was extracted. Then, the size of an esophagus lesion and the extent of esophagitis were observed. In Table 1, the esophagus lesion inhibition ratio (%) of a test group administered with the inventive test material, with respect to a control

group, is noted. FIG. 1 shows photographs of the effect of the inventive test material on esophagus mucous membrane.

As can be seen in Table 1, the Lonicerae Flos ethyl acetate fraction from among 5 fractions showed an esophagus injury inhibition ratio of 85% with respect to the control group, and showed an inhibition effect similar to 3,5-di-O-caffeoylquinic acid or 4,5-di-O-caffeoylquinic acid. It can be found that such an effect is much higher than that in oral administration of a Lonicerae Flos water extract according to a previous literature, and also is similar or greater compared to that in omeprazole used for a positive control group.

Table 1

[Table 1]

| [Table] | | |
|---|---|---|
| index | administraton amount (mg/kg) | esophagus lesion inhibition ratio with respect to control group (%) |
| control group | - | - |
| Comparative Example 1 (water extract) | 50 mg/kg | 30 |
| Example 1 (70% ethanol extract) | 50 mg/kg | 70 |
| Example 2 (ethanol extract) | 50 mg/kg | 70 |
| Example 3 (methanol extract) | 50 mg/kg | 68 |
| Example 4 (ethyle acetate fracton) | 50 mg/kg | 85 |
| Example 5 (butanol fraction) | 50 mg/kg | 75 |
| 3,5-di-CQA | 10 mg/kg | 90 |
| 4,5-di-CQA | 10 mg/kg | 81 |
| omeprazole | 10 mg/kg | 83 |

<Experimental Example 2: Measurement of lipid peroxide of esophagus tissue within a rat model of gastroesophageal reflux disease >

A test for finding out the inhibition of lipid peroxidation by the inventive Lonicerae Flos extract, the inventive Lonicerae Flos fraction, or a compound isolated from the fraction (3,5-di-O-caffeoylquinic acid or 4,5-di-O-caffeoylquinic acid) was carried out. the esophagus mucous membrane extracted in Experimental Example 1 was taken out, and added in 1 ml of tris-hydrochloric acid buffer solution (pH 7.0), followed by ultrasonic grinding. After centrifugation (600x g , 4°C) for 5 minutes, 0.3 mℓ of supernatant was added with 0.9mℓ of trichloroacetic acid (8%). Then, after centrifugation (10,000 × g , 4°C) for 5 minutes, 1mℓ of supernatant was added with 0.25 mℓ of TBA(1%), and then with 2mℓ of n-butanol. Then, after centrifugation (3,000 × g , 4°C) for 5 minutes, on 1 mℓ of butanol fraction, the absorbency was measured at 532 nm. As a reference material, malonaldehyde bis-dimethyl acetal was used. The result was expressed as nmol/mg protein, and protein quantitative analysis was carried out by Bradford assay. As a result, FIG. 2 shows the extent of lipid peroxidation of esophagus tissue in a rat model of gastroesophageal reflux disease, which is represented by malondialdehyde. From among Lonicerae Flos extracts containing a phenolic content, the group (Example 4) treated with ethyl acetate fraction showed a high lipid peroxidation inhibiting ratio of 46.2%. 3,5-di-O-caffeoylquinic acid and 4,5-di-O-caffeoylquinic acid showed lipid peroxidation inhibiting ratios of 49.1% and 50.2%, respectively. The group treated with omeprazole showed a lipid peroxidation inhibiting ratio of 53.9%.

<Experimental Example 3: TNF-alpha level analysis on an anti-inflammatory effect within esophagus tissue>

On the Lonicerae Flos extract, the Lonicerae Flos fraction, or a compound isolated from the fraction (3,5-di-O-caffeoylquinic acid or 4,5-di-O-caffeoylquinic acid), having a therapeutic effect in a rat model of gastroesophageal reflux disease, in order to measure an anti-inflammatory effect within esophagus tissue, a level of Tumor necrosis factor-alpha(TNF-α) playing an important role in inflammatory disease mechanism was measured within esophagus tissue of a rat (Nippon Rinsho, 68(5), 819-822, 2010). As a kit for this measurement, rat TNF-α ELISA kit (Cat No:88-7340, eBioscience) was used. Each of esophagus tissues of all test groups, obtained from <Experimental Example 1> was introduced into homogenizing buffer (10x) (pH 7.4: 1.15% KCl, 50 mM Tris-HCl, 1mM EDTA), followed by grinding by

a homogenizer. Then, after centrifugation at 3,000 rpm or more for 10 minutes, the homogenized tissue liquid (supernatant) was separated, and then stored in a deep freezer for the use in the experiment. After all antigen-antibody reactions were completed, the absorbency was measured by ELISA Reader (BIORAD) at 450 nm, and then the amount of TNF-$\alpha$ in esophagus tissue was expressed by using a reference curve of absorbency. The inflammation inhibition ratio (%) of a test material was calculated by the following equation. Also, the anti-inflammatory effect by the Lonicerae Flos extract in a rat model of gastroesophageal reflux disease is noted in Table 2.

$$\text{Inflammation inhibition ratio (\%)} = 1-((\text{test group}-\text{normal group})/(\text{control group}-\text{normal group})) \times 100$$

Table 2

[Table 2]

| [Table] | | | |
|---|---|---|---|
| index | amount ( mg/kg) | TNF-alpha (pg/mg Tissue weight) | inflammation inhibition ratio (%) |
| Normal group | - | 120.3 | - |
| Control group | - | 325.2 | - |
| Comparative Example 1 (water extract) | 50 | 270.3 | 26.8 |
| Example 1 (70% ethanol extract) | 50 | 220.3 | 51.2 |
| Example 4 (ethyle acetate fracton) | 50 | 132.5 | 94.0 |
| Example 5 (butanol fraction) | 50 | 178.3 | 71.7 |
| 3,5-di-CQA | 10 | 145.3 | 87.8 |
| 4,5-di-CQA | 10 | 150.1 | 85.5 |
| omeprazole | 10 | 160.2 | 80.5 |

As noted in Table 2, compared to a control group, all groups treated with the Lonicerae Flos extract showed a reduced TNF-alpha level. Especially, the ethyl acetate fraction (Example 4) showed the highest anti-inflammatory effect (inflammation inhibition ratio of 94.0%), which was better than that (80.5%) in the group treated with omeprazole alone. 3,5-di-O-caffeoylquinic acid and 4,5-di-O-caffeoylquinic acid showed high inflammation inhibition ratios of 87.8% and 85.5%, respectively.

<Experimental Example 4: the protective effect of esophagus mucous membrane by a Lonicerae Flos fraction>

On the Lonicerae Flos extract, the Lonicerae Flos fraction, or a compound isolated from the fraction (3,5-di-O-caffeoylquinic acid or 4,5-di-O-caffeoylquinic acid), having a therapeutic effect in a rat model of gastroesophageal reflux disease, the amounts of Hexosamine and Sialic acid constituting esophagus mucous membrane of esophagus tissue were measured.

1. Measurement of the amount of hexosamine in esophagus tissue

Esophagus tissues of all test groups, obtained from <Experimental Example 1>, were immersed in ethanol, and then left in acetone for 2 days, and in ether for 1 day, followed by cleaning and drying. The test sample was weighed, added with 5ml of 4N HCl solution, and hydrolyzed by being heated at 100 °C for 9 hours. Then, the resultant product was cooled at room temperature, and filtered. 0.5 mℓ of filtrate was added with 0.5ml of 4N NaOH solution for neutralization, and then added with 1ml of acetylacetone solution, followed by shaking. Then, the resultant product was heated at 100 °C for 20 minutes, and cooled, and then added with 5ml of isoamylalcohol, followed by shaking for 2 minutes. After centrifugation at 3,000 rpm for 10 minutes, 2ml of supernatant was collected. The supernatant was added with 0.5 mℓ of a color agent, and left for 15 minutes, and then its absorbency was measured at 530 nm. From a calibration curve

obtained by using Glucosamine, the content of hexosamine was calculated.

2. Measurement of the amount of Sialic acid in esophagus tissue

Esophagus tissues of all test groups, obtained from <Experimental Example 1>, were immersed in ethanol, and then left in acetone for 2 days, and in ether for 1 day, followed by cleaning and drying. The test sample was weighed, added with 5ml of 0.1N $H_2SO_4$ solution, and hydrolyzed by being heated at 80°C for 1 hour. Then, the resultant product was cooled at room temperature, and filtered. 0.2 mℓ of filtrate was mixed with 0.1ml of 0.2M periodate solution, and left at room temperature for 20 minutes. Then, the resultant solution was mixed with 1ml of 10% arsenite solution until a yellowish brown color disappeared. 3ml of 0.6% TBA solution was added thereto, followed by mixing. The resultant product was covered with a cap, and heated at 100 °C for 15 minutes, cooled, and left in a cool bath for 5 minutes, followed by centrifugation at 3,000 rpm for 10 minutes. A supernatant was collected, and its absorbency was measured at 549nm. From a calibration curve obtained by using N-acetylneuraminic acid, the content of Sialic acid was calculated.

The result of this test is noted in Table 3. A gastroesophageal reflux disease -induced group showed reduced contents of hexosamine and sialic acid compared to a normal group. Also, when administered with the Lonicerae Flos extract, the Lonicerae Flos fraction, 3,5-di-CQA and 4,5-di-CQA, the contents of hexosamine and sialic acid were significantly increased compared to that of a control group. This result indicates that the esophagus' mucous membrane's components reduced by gastroesophageal reflux disease are treated by the Lonicerae Flos extract, the Lonicerae Flos fraction, 3,5-di-CQA and 4,5-di-CQA while the esophagus' mucous membrane is returned to a normal state. A gastroesophageal reflux disease -induced rat showed a reduced amount of hexosamine. In consideration of the correlation between gastroesophageal reflux disease treatment and hexosamine within the mucous membrane, the change in hexosamine content in the esophagus tissue was in proportion to the extent of gastroesophageal reflux disease. This result indicates that the Lonicerae Flos extract or the Lonicerae Flos fraction quickly normalizes a clinically injured mucous membrane of the esophagus, and allows the esophagus tissue to defend against gastric acid, thereby reducing a relapse rate of gastroesophageal reflux disease.

Table 3

[Table 3]

| [Table] | | | |
|---|---|---|---|
| Group | Administra tion amount(mg/ kg) | unit: μg/100mg dry tissue | |
| | | Sialic acid | Hexosamine |
| Normal group | - | 125.2 | 1101.3 |
| Control group | - | 95.2 | 925.3 |
| Comparative Example 1 (Water extract) | 50 | 123.3 | 1130.5 |
| Example 1 (70% ethanol extract) | 50 | 138.0 | 1220.5 |
| Example 4 (ethyle acetate fraction) | 50 | 163.3 | 1512.5 |
| Example 5 (butanol fraction) | 50 | 147.8 | 1278.3 |
| 3,5-di-CQA | 10 | 155.3 | 1445.2 |
| 4,5-di-CQA | 10 | 148.1 | 1380.2 |
| omeprazole | 10 | 132.2 | 1176.3 |

<Experimental Example 5: HPLC analysis of a Lonicerae Flos fraction>

HPLC analysis conditions on the inventive Lonicerae Flos extract, its fraction, or the compound represented by Formula 1 or 2 are noted in Table 4 below.

Table 4

[Table 4]

| [Table] | |
|---|---|
| Instrument | Shiseido Nonospace SI-2 |

(continued)

| [Table] | | | |
|---|---|---|---|
| Column | Capcell Pak C18 UG80 4.6 × 150mm (5 μm) | | |
| UV wavelength | 254nm | | |
| Flow rate | 1.00 mL/mim | | |
| Injection volume | 5μL | | |
| Temperature | 40°C | | |
| Mobile phase | A) 5% Acetonitrile B) 80% Acetonitrile | | |
| Gradient program | Min | A (%) | B (%) |
| | 0 | 100 | 0 |
| | 10 | 95 | 5 |
| | 20 | 80 | 20 |
| | 25 | 75 | 25 |
| | 35 | 50 | 50 |
| | 40 | 100 | 0 |
| | 50 | 100 | 0 |

With respect to the inventive Lonicerae Flos extract and its fraction, the contents of 3,5-di-O-caffeoylquinic acid and 4,5-di-O-caffeoylquinic acid are noted in Table 5.

Table 5

[Table 5]

| [Table] | | |
|---|---|---|
| Index | Content of 3,5-di-CQA(%) | Content of 4,5-di-CQA(%) |
| Comparative Example 1 (Water extract) | 0.8 | 0.08 |
| Example 1 (70% ethanol extract) | 2.1 | 1.0 |
| Example 2 (ethanol extract) | 2.4 | 1.1 |
| Example 3 (methanol extract) | 2.2 | 1.2 |
| Example 4 (ethyle acetate fraction) | 13.0 | 2.5 |
| Example 5 (butanol fraction) | 6.7 | 1.1 |

As a result of HPLC analysis on the inventive Lonicerae Flos extract and its fraction, it was found that in a 70% ethanol extract, an ethanol extract, a methanol extract, a butanol fraction, and an ethyl acetate fraction, the content of 3,5-di-O-caffeoylquinic acid was 2% or more, and the content of 4,5-di-O-caffeoylquinic acid was 1% or more. In a gastroesophageal reflux disease -induced rat, the esophagus lesion inhibition ratio of these Lonicerae Flos fractions was higher than that of a control group, by 60% or more. In an ethyl acetate fraction showing the same esophagitis inhibiting effect as the compound 1 or 2, 3,5-di-O-caffeoylquinic acid was 10% or more, and 4,5-di-O-caffeoylquinic acid was 2% or more. Thus, it can be found that the ethyl acetate fraction is better in view of a cost or an effect in treatment of gastroesophageal reflux disease, compared to separation and purification of the compound 1 or 2.

<Experimental Example 6: toxicity test of acute oral administration of a Lonicerae Flos fraction>

By applying the Lonicerae Flos extract of Example 1, fractions of Examples 4 and 5, and 3,5-di-O-caffeoylquinic acid and 4,5-di-O-caffeoylquinic acid from Example 6, to SPF (specific pathogen free) SD-based female/male rats aged 6 weeks, an acute toxicity test was carried out.

Female 5 rats and male 5 rats of each test group were orally administered with the test materials from Examples

in a dose of 1.0 g/kg or 2.0 g/kg. The test materials were suspended in 0.5% CMC (Carboxymethyl cellulose) solution before the administration. When the test materials were administered, mortality, clinical symptoms, and weight changes of animals were observed. 7 days after the administration, through autopsy, a hematologic examination and a blood chemical test were carried out. Then, with the naked eye, it was observed if there was abnormality in abdominal cavity organs and thoracic cage organs. As a result, from among all animals administered with the test materials, not one showed an unusual clinical symptom or had died due to the administered compounds. Also, through weight measurement, a blood test, a blood chemical test, and autopsy results, an abnormal change was not observed. As a result, it was found that the inventive Lonicerae Flos extract, its fraction, or the compound 1 or 2 isolated from the fraction are safe materials not showing toxicity in a rat as long as these materials are used in an amount of up to 2.0 g/kg.

From the experiments, it was found that the inventive Lonicerae Flos extract, its fraction, or the compound represented by Formula 1 or 2 is highly effective in treatment of gastroesophageal reflux disease, and shows a high anti-oxidant effect and a high anti-inflammatory effect in the esophagus' mucous membrane. Also, these materials show a good effect in regeneration and protection of the esophagus' mucous membrane, and thus may be usefully and safely utilized for drugs and health care foods for treatment or prevention of gastroesophageal reflux disease.

**Brief Description of Drawings**

The foregoing and other objects, features and advantages of the present invention will become more apparent from the following detailed description when taken in conjunction with the accompanying drawings in which:

FIG. 1 shows photographs of an esophageal lesion on an esophagus' mucous membrane of rats in a control group, and a test group in a rat model of acute gastroesophageal reflux disease, wherein rats in the test group were orally administered with a Lonicerae Flos water extract, a 70% ethanol extract, a butanol fraction, and an ethyl acetate fraction, in an amount of 50 mg/kg (each), and with 3,5-di-O-caffeoylquinic acid, 4,5-di-O-caffeoylquinic acid, and omeprazole in an amount of 10 mg/kg (each); and

FIG. 2 shows the extent of lipid peroxidation (measured by malondialdehyde) in the esophagus' mucous membrane of rats in a control group, and a test group in a rat model of acute gastroesophageal reflux disease, wherein rats in the test group were administered with a Lonicerae Flos water extract, a 70% ethanol extract (Example 1), an ethyl acetate fraction (Example 4), a butanol fraction (Example 5), 3,5-di-O-caffeoylquinic acid, 4,5-di-O-caffeoylquinic acid, and omeprazole.

**Mode for the Invention**

Hereinafter, the present invention will be described in detail with reference to Examples. However, the following examples are only for illustrative purposes.

<Example 1> Preparation of 70% ethanol aqueous solution extract of Lonicerae Flos

Lonicerae Flos used in the present invention was a dried bud of Lonicerae Flos. Powder obtained by grinding Lonicerae Flos was sliced into an appropriate size, and then introduced in an amount of 1kg into an extraction vessel. 10L of 70% ethanol aqueous solution solvent was added thereto, followed by stirring and extraction at 60°C for 6 hours. The resultant product was filtered by filter paper, so as to provide an extract. The extracting step was repeated three times. Then, through vacuum-concentration and drying of the solvent, 300g of 70% ethanol aqueous solution extract was obtained.

<Examples 2 to 3> Preparation of alcohol extract of Lonicerae Flos

320g of ethanol extract and 340g of methanol extract were obtained in the same manner as described in Example 1, except that ethanol and methanol were used, respectively, instead of the 70% ethanol aqueous solution extraction solvent in Example 1.

<Example 4> Preparation of ethyl acetate fraction of Lonicerae Flos extract

The 70% ethanol aqueous solution extract from Example 1 was added with 3L of water, and suspended. Then, 3L of ethyl acetate was added thereto, and extraction was repeated three times. Through vacuum filtration by filter paper, an ethyl acetate extract was obtained, and its solvent was removed. Then, as a residue, 30g of ethyl acetate fraction was obtained.

<Example 5> Preparation of butanol fraction of Lonicerae Flos extract

The 70% ethanol aqueous solution extract from Example 1 was added with 3L of water, and suspended. Then, 3L of butanol was added thereto, and extraction was repeated three times. Through vacuum filtration by filter paper, a butanol extract was obtained, and its solvent was removed. Then, as a residue, 75g of butanol fraction was obtained.

<Example 6> Preparation of a phenolic compound from Lonicerae Flos fraction (reference example)

(Example 6-1) Preparation of 3,5-di-O-caffeoylquinic acid

The ethyl acetate fraction obtained from Example 4 was purified with chromatography by using a HP-20 column (mobile phase: 100% ethanol), and then sub-fractions 1 to 4 were obtained. From among the fractions, fraction 3 was dissolved in ethanol, and purified with high-performance liquid chromatography so as to provide 3,5-di-O-caffeoylquinic acid. Herein, as a mobile phase, a mixed solvent of water and acetonitrile was used, with a concentration gradient of acetonitrile (sequential polarity of from 0 to 20 vol%) for 50 minutes. The flow rate was 10 mL/min, and Capcell pak UG120 ($6.0\times50$mm, 5$\mu$m) column was used.

3,5-di-O-caffeoylquinic acid: $^1$H-NMR($CD_3OD$) $\delta$ 2.11-2.34(4H, m, H-2,6), 3.95(1H, dd, J=3.0, 7.8Hz, H-4), 5.37-5.44(2H, m, H-3,5), 6.26, 6.36(1H each, J=15.9Hz, H-8', 8"), 6.77(2H, d, J=7.8Hz, H-5', 5"), 6.96(2H, dd, J=2.1, 8.1Hz, H-6', 6"), 7.05, 7.06(1H each, d, J=2.1Hz, H-2', 2"), 7.57, 7.61(1H each, d, J=15.9Hz, H-7', 7")

$^{13}$C-NMR(125MHz, $CD_3OD$) $\delta$ 36.3(C-2), 38.2(C-6), 71.1(C-4), 72.0(C-5), 72.9(C-3), 75.2(C-1), 115.1, 115.2(C2', 2"), 115.3, 115.5(C-8', 8"), 116.5(C-5', 5"), 123.0, 123.1(C-6', 6"), 127.7, 127.8(C-1', 1"), 146.6(C-3', 3"), 147.0, 147.2(C-7', 7"), 149.3, 149.4(C-4', 4"), 168.5, 168.9(C-9', 9"), 178.1(COOH)

(Example 6-2) Preparation of 4,5-di-O-caffeoylquinic acid

The ethyl acetate fraction obtained from Example 4 was purified with chromatography by using a HP-20 column (mobile phase: 100% ethanol), and then sub-fractions 1 to 4 were obtained. From among the fractions, fraction 3 was dissolved in ethanol, and purified with high-performance liquid chromatography so as to provide 4,5-di-O-caffeoylquinic acid. Herein, as a mobile phase, a mixed solvent of water and acetonitrile was used, with a concentration gradient of acetonitrile (sequential polarity of from 0 to 20 vol%) for 50 minutes. The flow rate was 10 mL/min, and Capcell pak UG120 (6.0 150mm, 5$\mu$m) column was used.

4,5-di-O-caffeoylquinic acid: $^1$H-NMR (500MHz, $CD_3OD$) $\delta$ 1.98-2.30(4H, m, H-2,6), 4.33(1H, br s, H-3), 5.10(1H, dd, J=2.6, 9.5Hz, H-4), 5.65(1H, m, H-5), 6.19, 6.27(1H each, d, J=15.9Hz, H-8', 8"), 6.72, 6.73(1H each, d, J=8.1Hz, H-5', 5"), 6.88, 6.90(1H each, dd, J=1.8, 8.1Hz, H-6', 6"), 6.99, 7.01(1H each, d, J=1.8Hz, H-2', 2"), 7.50, 7.58(1H each, d, J=15.9Hz, H-7', 7")

$^{13}$C-NMR(125MHz, $CD_3OD$) $\delta$ 76.1(C-1), 38.3(C-2), 69.8(C-3), 75.8(C-4), 69.0(C-5), 39.8(C-6), 127.3, 127.4(C-1', 1"), 146.5(C-3', 3"), 149.4(C-4', 4"), 116.1(,C-5', 5"), 122.9(C-6', 6"), 147.2, 147.3(C-7', 7"), 114.4(C-8', 8"), 168.1, 168.3(C-9', 9")

<Example 7> Preparation of pharmaceutical formulation

(Example 7-1) Preparation of powder of Lonicerae Flos ethyl acetate fraction

Lonicerae Flos ethyl acetate fraction 20 mg
lactose 100 mg
talc 10 mg
The ingredients above are mixed and filled into an airtight bag so as to provide a powder.

(Example 7-2) Preparation of tablet

Lonicerae Flos ethyl acetate fraction 10 mg
Corn starch 100 mg
lactose 100 mg
magnesium stearate 2 mg

The ingredients above are mixed and tabletted by a conventional tablet preparation method so as to provide a tablet.

(Example 7-3) Preparation of capsule

Lonicerae Flos ethyl acetate fraction 10 mg
Crystalline cellulose 30 mg
lactose 14.8 mg
magnesium stearate 0.2 mg

The ingredients above are mixed and filled into a gelatin capsule according to a conventional capsule preparation method so as to provide a capsule.

(Example 7-4) Preparation of injection

Lonicerae Flos ethyl acetate fraction 10 mg
mannitol 180 mg
sterilized distilled water for injection 2974 mg
$Na_2HPO_4$,$12H_2O$ 26mg

According to a conventional injection preparation method, an injection is prepared by including the contents above per an ample (2 mℓ).

(Example 7-5) Preparation of liquid

Lonicerae Flos ethyl acetate fraction 20 mg
Isomerized glucose syrup 10 g
mannitol 5 g
pure water proper quantity

According to a conventional liquid preparation method, respective ingredients are added in pure water, and dissolved. Then, lemon fragrance is added thereto in a proper quantity, and the ingredients are mixed. Then, pure water is added so that the total volume can be 100 mℓ. The mixture is filled into a brown bottle and sterilized.

<Example 8> Preparation of a health care food

(Example 8-1) Preparation of drink

honey 522 mg
thioctic acid amide 5 mg
nicotinamide 10 mg
riboflavin sodium hydrochloride 3 mg
pyridoxine hydrochloride 3 mg
inositol 30 mg
orotic acid 50 mg
Lonicerae Flos ethyl acetate fraction 100 mg
Pure water 200 mL

A drink is prepared by the compositions and contents above according to a conventional method.

(Example 8-2) vitamin-mixed formulation

Lonicerae Flos ethyl acetate fraction 100 mg
Phenolic acid compound 100 mg
vitamin A acetate 70 $\mu$g
vitamin E 1.0 mg
vitamin B1 0.13 mg
vitamin B2 0.15 mg
vitamin B6 0.2 $\mu$g
vitamin C 10 mg
biotin 10 $\mu$g
nicotinic acid amide 1.7 mg
folic acid 50 $\mu$g
calcium pantothenate 0.5 mg
zinc oxide 0.82 mg
magnesium carbonate 25.3 mg
potassium dihydrogen phosphate 15 mg
potassium citrate 90 mg
calcium carbonate 100 mg magnesium chloride 24.8 mg

As a preferred embodiment, the mixture of vitamins and minerals has a composition ratio relatively appropriate for a health care food. However, the composition ratio may be freely changed. In other words, the above ingredients may be mixed and prepared as a granule according to a conventional health care food preparation method, and then may be used for preparation of a health care food composition according to a conventional method.

## Claims

1. A pharmaceutical composition for use in treating or preventing gastroesophageal reflux disease, the composition comprising, as active ingredients, 3,5-di-O-caffeoylquinic acid (Formula 1) and 4,5-di-O-caffeoylquinic acid (Formula 2) as a C1-C4 alcohol extract or a 50-100% C1-C4 alcohol aqueous solution extract of Lonicerae Flos, or as a ethyl acetate or butanol fraction of said extract,

[Formula 1]

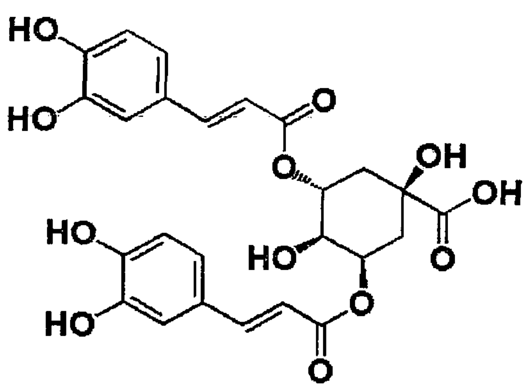

[Formula 2]

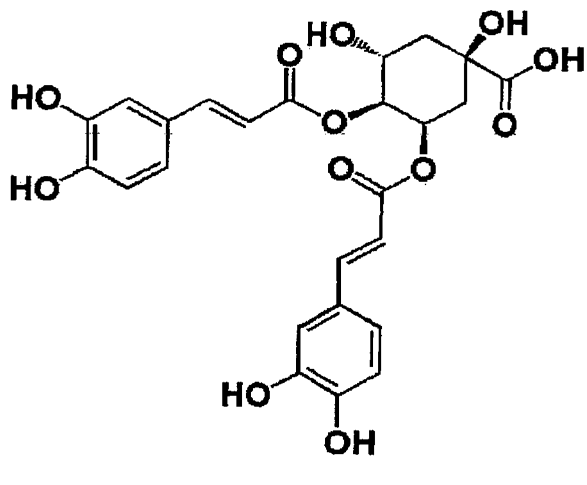

and wherein in the composition the content of 3,5-di-O-caffeoylquinic acid (Formula 1) is 2% or more in relation to the extract or fraction and the content of 4,5-di-O-caffeoylquinic acid (Formula 2) is 1% or more in relation to the extract or fraction.

2. The pharmaceutical composition for the use as claimed in claim 1, further comprising at least one therapeutic agent

of gastroesophageal reflux disease selected from the group consisting of proton-pump inhibitors and histamine-2 receptor antagonists.

3. A health care food composition for use in improving or preventing gastroesophageal reflux disease, the composition comprising, as active ingredients, 3,5-di-O-caffeoylquinic acid (Formula 1) and 4,5-di-O-caffeoylquinic acid (Formula 2) as a C1-C4 alcohol extract or a 50-100% C1-C4 alcohol aqueous solution extract of Lonicerae Flos, or as a ethyl acetate or butanol fraction of said extract,

[Formula 1]

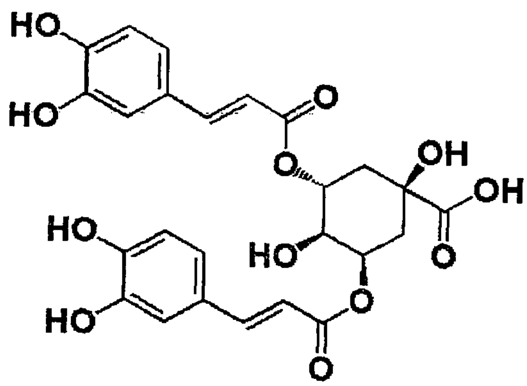

[Formula 2]

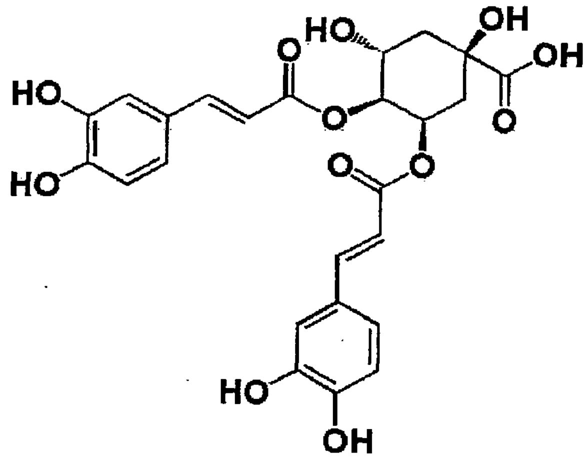

and wherein in the composition the content of 3,5-di-O-caffeoylquinic acid (Formula 1) is 2% or more in relation to the extract or fraction and the content of 4,5-di-O-caffeoylquinic acid (Formula 2) is 1% or more in relation to the extract or fraction.

**Patentansprüche**

1. Pharmazeutische Zusammensetzung zur Verwendung in der Behandlung oder Prävention von Refluxösophagitis, wobei die Zusammensetzung als aktive Bestandteile 3,5-Di-O-caffeoylchininsäure (Formel 1) und 4,5-Di-O-caffeoylchininsäure (Formel 2) als einen Cl-C4-Alkoholextrakt oder einen 50-100% C1-C4-Alkohol-wässrige-Lösung-Extrakt von Lonicerae flos oder als eine Ethylacetat- oder Butanolfraktion des Extrakts umfasst,

[Formel 1]

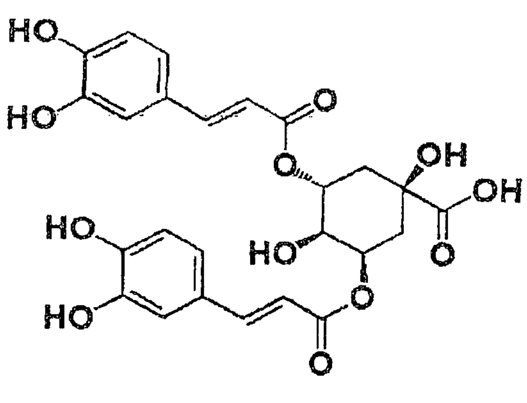

[Formel 2]

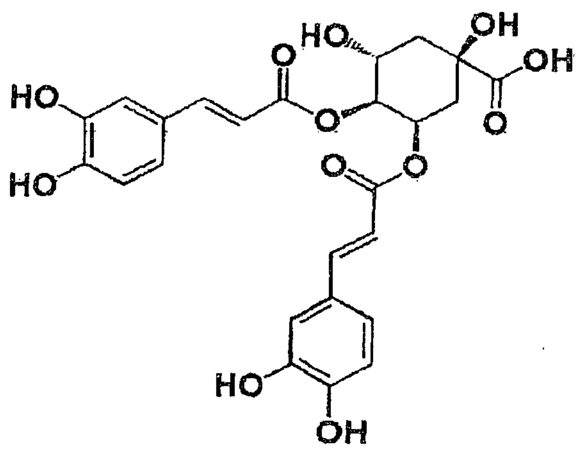

und wobei in der Zusammensetzung der Gehalt von 3,5-Di-O-caffeoylchininsäure (Formel 1) 2% oder mehr im Verhältnis zu dem Extrakt oder der Fraktion beträgt und der Gehalt von 4,5-Di-O-caffeoylchininsäure (Formel 2) 1% oder mehr im Verhältnis zu dem Extrakt oder der Fraktion beträgt.

**2.** Pharmazeutische Zusammensetzung zur Verwendung wie in Anspruch 1 beansprucht, ferner umfassend mindestens ein therapeutisches Agens der Refluxösophagitis, ausgewählt aus der Gruppe, bestehend aus Protonenpumpeninhibitoren und Histamin-2-Rezeptorantagonisten.

**3.** Gesundheitsnahrungsmittelzusammensetzung zur Verwendung in der Verbesserung oder Prävention von Refluxösophagitis, wobei die Zusammensetzung als aktive Bestandteile 3,5-Di-O-caffeoylchininsäure (Formel 1) und 4,5-Di-O-caffeoylchininsäure (Formel 2) als C1-C4-Alkoholextrakt oder als einen 50-100% C1-C4-Alkohol-wässrige-Lösung-Extrakt von Lonicerae flos oder als eine Ethylacetat- oder Butanolfraktion des Extrakts umfasst,

[Formel 1]

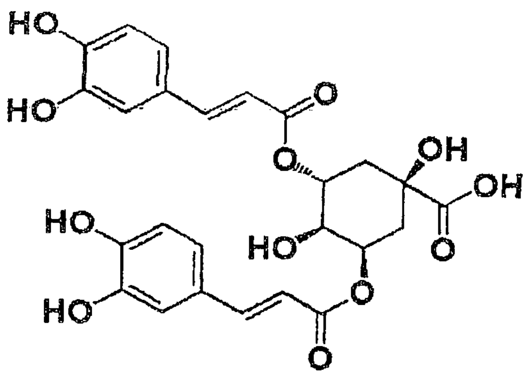

[Formel 2]

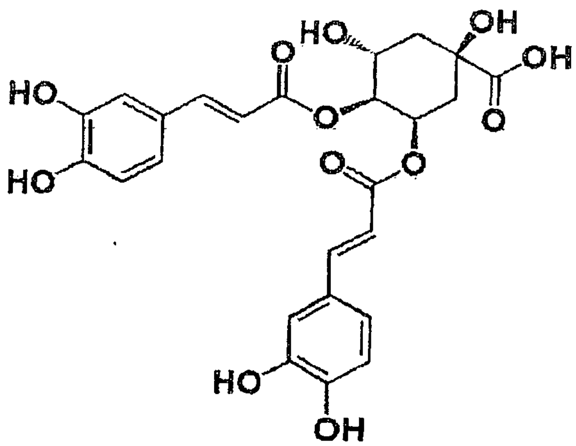

und wobei in der Zusammensetzung der Gehalt von 3,5-Di-O-caffeoylchininsäure (Formel 1) 2% oder mehr im Verhältnis zu dem Extrakt oder der Fraktion beträgt und der Gehalt von 4,5-Di-O-caffeoylchininsäure (Formel 2) 1% oder mehr im Verhältnis zu dem Extrakt oder der Fraktion beträgt.

**Revendications**

**1.** Composition pharmaceutique pour l'utilisation dans le traitement et la prévention de la maladie de reflux gastrooesophagien, la composition comprenant, comme ingrédients actifs, l'acide 3,5-di-O-caféoylquinique (formule 1) et l'acide 4,5-di-O-caféoylquinique (formule 2) en tant qu'un extrait d'alcool en $C_1$-$C_4$ de Lonicerae flos ou d'un extrait de Lonicerae flos obtenu avec une solution aqueuse d'alcool à 50-100% ou en tant qu'une fraction d'acétate d'éthyle ou de butanol dudit extrait

[Formula 1]

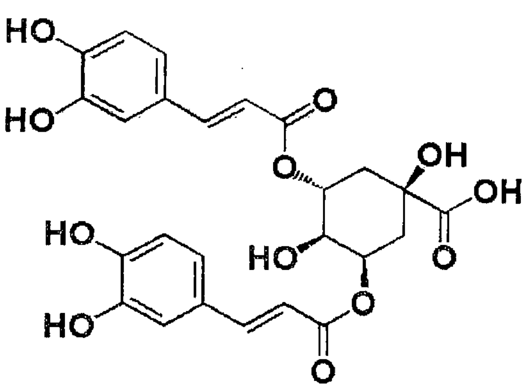

[Formula 2]

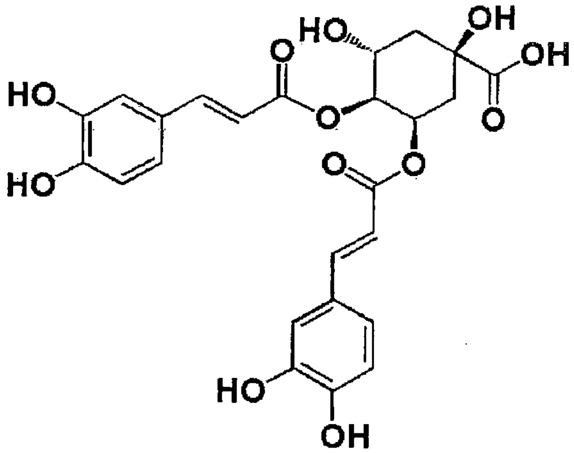

où, dans la composition, la teneur en acide 3,5-di-O-caféoylquinique (formule 1) est de 2% ou plus par rapport à

l'extrait ou la fraction, et la teneur en acide 4,5-di-O-caféoylquinique (formule 2) est de 1% ou plus par rapport à l'extrait ou la fraction.

2. Composition pharmaceutique pour l'utilisation selon la revendication 1, comprenant en outre au moins un agent thérapeutique de la maladie de reflux gastro-oesophagien choisi parmi le groupe consistant en inhibiteurs de la pompe à protons et antagonistes du récepteur histaminique 2.

3. Composition d'aliments de santé pour l'utilisation dans l'amélioration ou la prévention de la maladie de reflux gastro-oesophagien, la composition comprenant, comme ingrédients actifs, l'acide 3,5-di-O-caféoylquinique (formule 1) et l'acide 4,5-di-O-caféoylquinique (formule 2) en tant qu'un extrait d'alcool en $C_1$-$C_4$ de Lonicerae flos ou d'un extrait de Lonicerae flos obtenu avec une solution aqueuse d'alcool à 50-100% ou en tant qu'une fraction d'acétate d'éthyle ou de butanol dudit extrait

[Formula 1]

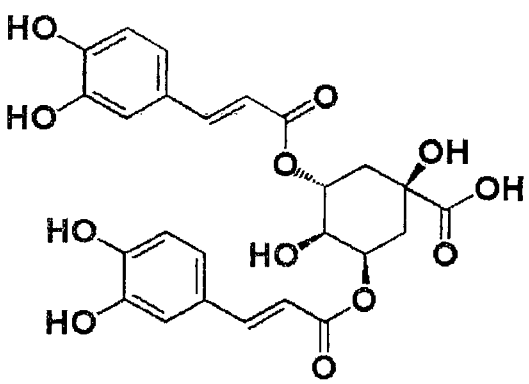

[Formula 2]

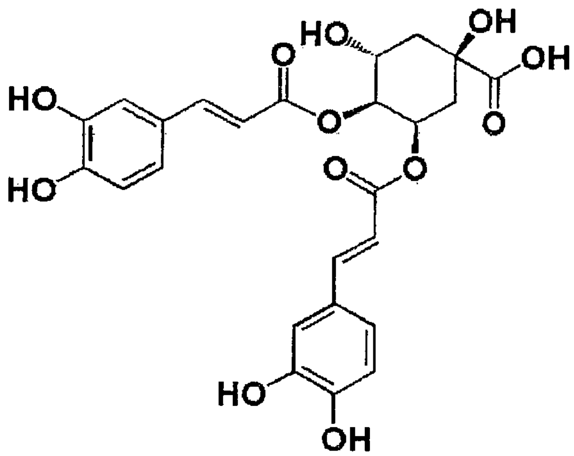

où, dans la composition, la teneur en acide 3,5-di-O-caféoylquinique (formule 1) est de 2% ou plus par rapport à l'extrait ou la fraction, et la teneur en acide 4,5-di-O-caféoylquinique (formule 2) est de 1% ou plus par rapport à l'extrait ou la fraction.

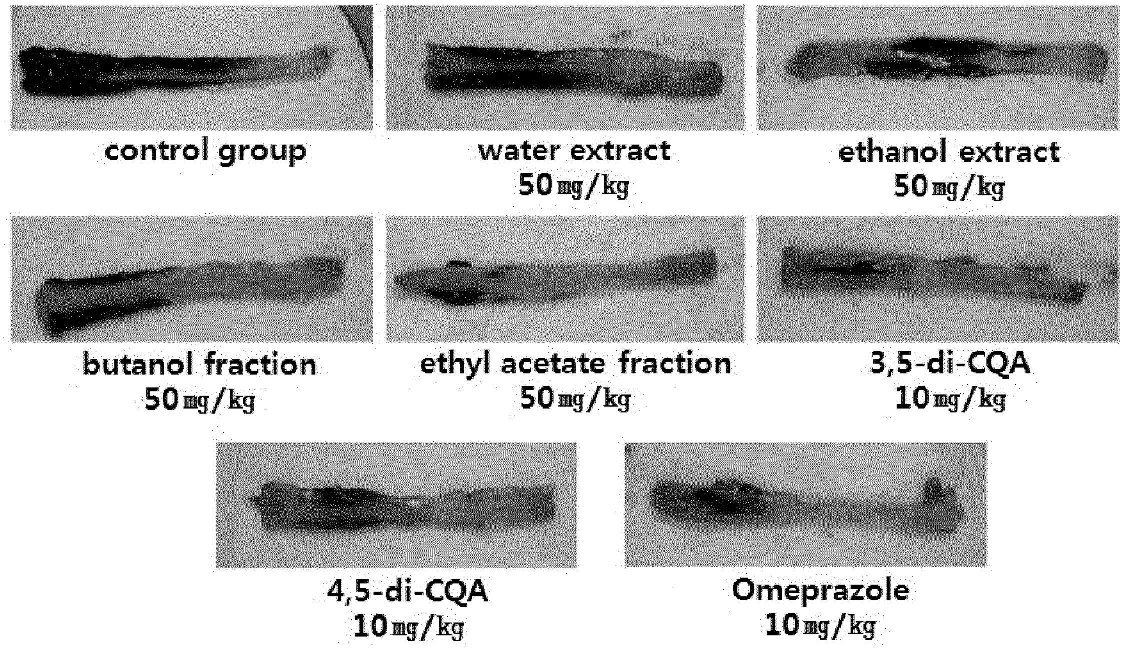
control group
water extract
50㎎/㎏
ethanol extract
50㎎/㎏
butanol fraction
50㎎/㎏
ethyl acetate fraction
50㎎/㎏
3,5-di-CQA
10㎎/㎏
4,5-di-CQA
10㎎/㎏
Omeprazole
10㎎/㎏

Fig. 1

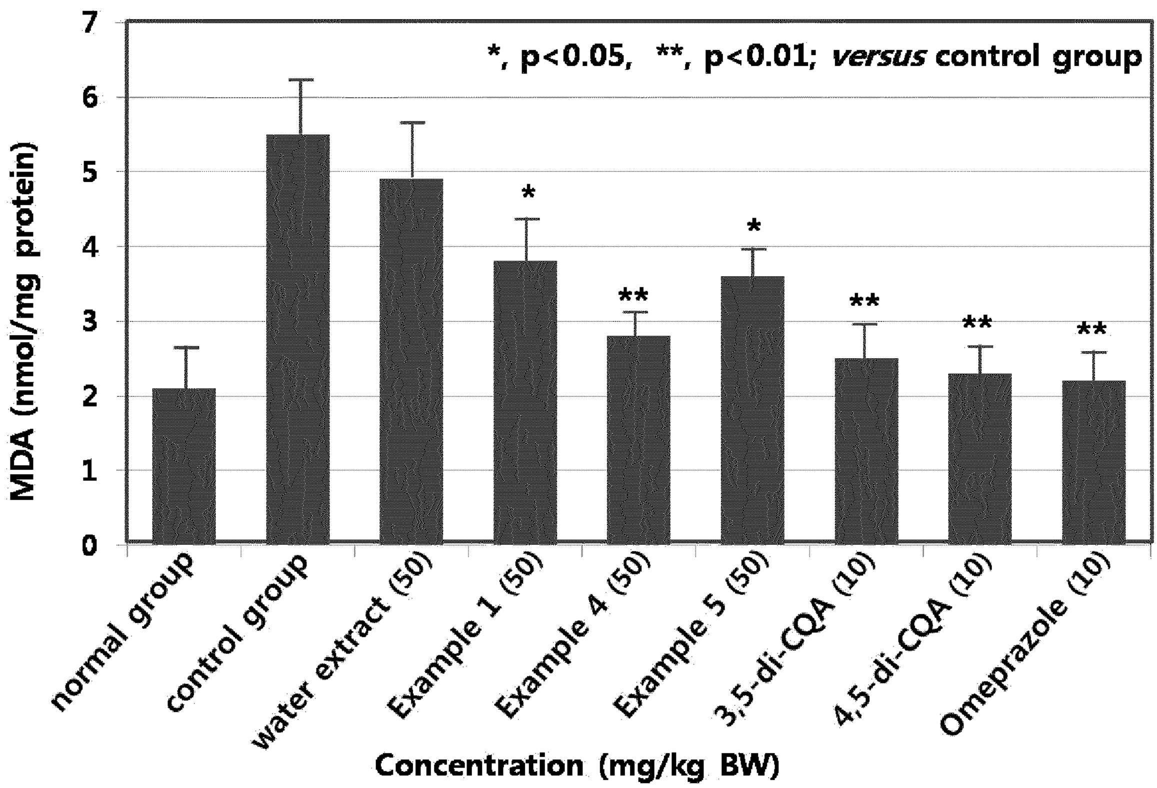
*, p<0.05, **, p<0.01; *versus* control group
MDA (nmol/mg protein)
0
1
2
3
4
5
6
7
*
**
*
**
**
**
normal group
control group
water extract (50)
Example 1 (50)
Example 4 (50)
Example 5 (50)
3,5-di-CQA (10)
4,5-di-CQA (10)
Omeprazole (10)
Concentration (mg/kg BW)

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 100878436


### Non-patent literature cited in the description


- **LEE, S. J.** *Arch. Pharm. Res.,* 1993, vol. 16, 25
- **S. J. LEE.** *Phytotherapy research,* 1998, vol. 12, 445-447
- **TAE, J ; HAN.** *Clin Chim Acta,* vol. 330 (1-2), 165-171, 2003
- **KU, S. K.** *World J Gastroenterol.,* 2009, vol. 15 (38), 4799-4805
- *J. Sep. Sci.,* 2008, vol. 31, 3519-3526
- **NAKAMURA K et al.** *Jpn. J. Pharmacol.,* 1982, vol. 32 (3), 445-456
- **OKABE S et al.** *Jpn. J. Pharmacol.,* 1995, vol. 69 (4), 317-323
- *Nippon Rinsho,* 2010, vol. 68 (5), 819-822